# EUROPEAN PATENT APPLICATION

(11) **EP 1 384 721 A1**
(43) Date of publication of application: **28.01.2004**
(21) Application number: 03254626.9
(22) Date of filing: 23.07.2003
(51) Int. Cl.: C07D 407/06, A61K 31/351, A61P 31/04

(54) **Stable amorphous calcium pseudomonate and processes for the preparation thereof**

(30) Priority: 25.07.2002 IL 15090702
(71) Applicant: CHEMAGIS LTD., Tel Aviv 61090 (IL)
(72) Inventor: Weisman, Alexander, Kiryat-Ekron 70500 (IL); Kaspi, Joseph, Givatayim 53201 (IL); Cherkez, Stephen, Caesarea 38900 (IL)
(74) Representative: Tubby, David George

(57) **Abstract**

The invention provides stable amorphous calcium pseudomonate.

## Description

The present invention relates to stable amorphous calcium pseudomonate, processes for the preparation thereof and pharmaceutical compositions containing the same.

Pseudomonic acid A (hereinafter referred as pseudomonic acid) known also as mupirocin is isolated from fermentation broth of Pseudomonas Fluorescens. This compound is a highly potent antibiotic and it is used topically for treatment of bacterial infections. Bactroban® ointment, in which the active ingredient is pseudomonic acid, was introduced in the US market in 1987.

Patents GB 1,577,545 and GB 1,577,730 mention non-toxic salts of pseudomonic acid as candidates for administration. Among them is the calcium salt. No data regarding this material or processes for its preparation were given.

Several years later US 4,916,155, US 5,191,093 and US 5,436,266 describe calcium pseudomonate. These patents describe crystalline calcium pseudomonate as a dihydrate and as an anhydrous material. A process for the preparation of crystalline calcium pseudomonate is also given. It is used for the treatment of bacterial infections formulated as a nasal ointment and as a topical cream. A nasal ointment was introduced to the US market in 1995 and a topical cream in 1997. These patents also teach that while amorphous calcium pseudomonate is thermally labile, and decomposes on storage, the crystalline forms are stable and are suitable for use in pharmaceutical preparations. One of these patents (4,916,155) claims the crystalline anhydrous calcium pseudomonate, another (US 5,191,093) claims a process for the preparation of crystalline calcium pseudomonate both hydrous and anhydrous, while the third (5,436,266) claims crystalline hydrates (specifically the dihydrate) of calcium pseudomonate.

The recent discovery of the usefulness of amorphous calcium pseudomonate in topical preparations described in EP application 1,174,133 indicated the necessity of obtaining a stable amorphous calcium pseudomonate.

Amorphous calcium pseudomonate was described in U.S. patents U.S. 4,916,155, U.S. 5,191,093 and U.S. 5,436,266. A process for its preparation is also described there. These patents claim crystalline calcium pseudomonate as a dihydrate or an anhydrous material. Amorphous calcium pseudomonate is also described, but not claimed there.

These patents state that amorphous calcium pseudomonate is not stable. Thus, the table in U.S. 4,916,155 (at the bottom of column 8 and the top of column 9, right after "Description 1") presents the following data:
Pseudomonic acid and calcium pseudomonate dehydrate have an initial purity that is -2% higher than amorphous calcium pseudomonate. Upon storage (especially at elevated temperatures) amorphous calcium pseudomonate decomposes much faster than crystalline calcium pseudomonate dihydrate. For example, after storage at 37°C for 2 weeks, the crystalline calcium pseudomonate did not change (reported 100.2% of initial purity), while amorphous calcium pseudomonate showed only 97.7% purity. Higher decomposition rates were shown at 50°C after 2 weeks storage: 98.7% for the crystalline and 80.1% for the amorphous calcium pseudomonate.

Moreover, the substances described in U.S. 4,916,155 cannot be used for therapeutical purposes. Mupirocin (pseudomonic acid), being a drug, is controlled for its purity. Thus, the British Pharmacopoeia states that mupirocin must have a minimal purity of 93%. The same is required for the calcium salt. The fact that the amorphous calcium pseudomonate described in the patent has an even lower quality than the starting material (pseudomonic acid) raises the question whether amorphous calcium pseudomonate of purity higher than 93%, suitable for pharmaceutical use, can be obtained according to the art taught in U.S. 4,916,155.

Process for the preparation of amorphous calcium pseudomonate is described in US 4,916,155, US 5,191,093 and US 5,436,266. A solution of pseudomonic acid is dissolved in a mixture of methanol and water and neutralized to pH 7.1 with calcium oxide. The solution is filtered and the solvents are evaporated under reduced pressure to give solid foam. Trituration of the foam with diethyl ether affords amorphous calcium pseudomonate.

This procedure is not applicable for a large-scale preparation. While handling a solid foam (that is usually rather sticky) is not a big problem in a laboratory scale of grams, it turns to be a huge, almost impossible task in large commercial scale. This process also uses diethyl ether in order to modify the foam obtained to a powder. Such an operation is also extremely difficult to carry out. Besides, this solvent is very inflammable and tends to make highly explosive mixtures with air. Therefore, its use in a large scale is a serious hazard.

When we tried to repeat the process as described in these patents we found out that besides the above-mentioned problems, an appreciable decomposition of the pseudomonate took place during the evaporation of the solvents. This decomposition occurred regardless of the conditions used to distill out the water.

Surprisingly it has now been found that lyophilization of a frozen solution of calcium pseudomonate afforded the amorphous calcium salt as a powdery material. The material also proved to be stable. It did not show decomposition when stored at an appropriate temperature.

More specifically, it has been found that neutralization of a suspension of pseudomonic acid in water with calcium hydroxide and lyophilization of the obtained solution results in a powder of amorphous calcium pseudomonate. The material thus obtained is stable and is suitable for use in pharmaceutical preparations (topical, nasal, otic, ophthalmic etc.).

Thus according to the present invention, there is now provided for the first time a stable amorphous calcium pseudomonate.

The invention also provides a process for the preparation of stable amorphous calcium pseudomonate comprising lyophilizing a frozen solution of calcium pseudomonate in water.

In preferred embodiments of the present invention said process is further characterized in that the solution of calcium pseudomonate in water is prepared by suspending pseudomonic acid in water and gradually adding calcium hydroxide to pH of 7.8-8.5, optionally filtering the solution from some insoluble material.

In especially preferred embodiments of the present invention the pH is maintained below 8.5 and the addition of calcium hydroxide is carried out at a temperature of below 15°C.

The invention also provides a stable pharmaceutical composition comprising amorphous calcium pseudomonate in a combination with a pharmaceutically acceptable carrier or excipient.

The following results illustrate findings according to the present invention:

**Table 1:**

| **Stability results of amorphous calcium pseudomonate** | | | | | | |
|---|---|---|---|---|---|---|
| Storage time | Related Substances (from fermentation process) | | | | Decomposition Products | |
| | P.A. B | P.A. C | P.A. D | P.A. E | D.P. I | D.P. II |
| t=0 | 0.10 | 0.09 | 0.90 | 0.10 | 0.44 | 1.00 |
| t=18 months (at 2-8°C) | 0.18 | 0.08 | 0.97 | 0.09 | 0.62 | 1.06 |
| Legend: P.A. = pseudomonic acid D.P. = decomposition product | | | | | | |

Samples thus prepared also remained amorphous during the 18 months storage period and did not show any evidence for spontaneous crystallization.

In addition, it has now been found that employing the process of the present invention results in no decomposition of the pseudomonic acid during the preparation of the calcium salt. Table 2 gives the results.

**Table 2:**

| **Decomposition products during the conversion of pseudomonic acid to amorphous calcium pseudomonate** | | | | | | |
|---|---|---|---|---|---|---|
| Sample | Related Substances (from fermentation process) | | | | Decomposition Products | |
| | P.A. B | P.A. C | P.A. D | P.A. E | D.P. I | D.P. II |
| Starting material | 0.57 | 0.16 | 1.17 | 0.21 | 0.58 | 0.81 |
| Ca salt made from | 0.40 | 0.10 | 1.25 | 0.15 | 0.62 | 1.10 |
| Note: look for legend and note under table 1. | | | | | | |

The calcium pseudomonate solution was prepared by a slow addition of solid calcium hydroxide to a suspension of pseudomonic acid in water. The calcium salt formed is gradually dissolved. The almost clear solution is filtered and frozen.

The use of calcium hydroxide is another feature of the invention. All the material used (pseudomonic acid, calcium hydroxide and water) are approved for use in human drugs. All the materials used have pharmacopoeial monographs. In this way the quality of the amorphous calcium pseudomonate is assured.

The safety (avoiding use of inflammable and explosive organic solvents) of the process and the ability to carry it out in a large commercial scale is still another feature of the invention.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Example

Pseudomonic acid (100 gr.) is suspended in water (1000 ml). The mixture is cooled below 15°C and calcium hydroxide (ca. 7.0 gr.) is added gradually until the pH is 8. The slightly turbid mixture is filtered and the solution is frozen and lyophilized. The product obtained is crushed and milled to give amorphous calcium pseudomonate in quantitative yield. The product was shown to be amorphous by XRD and IR analysis.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. Stable amorphous calcium pseudomonate.

2. Stable amorphous calcium pseudomonate having a purity of 93% or more.

3. Stable amorphous calcium pseudomonate having a purity of 96% or more.

4. A process for the preparation of stable amorphous calcium pseudomonate, as claimed in claims 1 to 3, comprising lyophilizing a frozen solution of calcium pseudomonate in water.

5. A process for the preparation of stable amorphous calcium pseudomonate, as claimed in claim 4, further **characterized that** the solution of calcium pseudomonate in water is prepared by suspending pseudomonic acid in water and gradually adding calcium hydroxide to pH 7.8-8.5 and optionally filtering the solution from insoluble material.

6. A process according to claim 4, where the pH is maintained below 8.5.

7. A process, according to claim 4, where the addition of calcium hydroxide is done below 15°.

8. A stable, amorphous calcium pseudomonate whenever prepared according to the process, as claimed in claim 4.

9. A stable pharmaceutical composition comprising amorphous calcium pseudomonate with a pharmaceutically acceptable carrier or excipients.

10. A stable pharmaceutical composition comprising amorphous calcium pseudomonate prepared from amorphous calcium pseudomonate, in combination with a pharmaceutically acceptable carrier or excipients.

11. A stable pharmaceutical composition according to claim 10 wherein the carrier is a cream.

12. Amorphous calcium pseudomonate comprising at least 96% pesudomonic acid A in the anionic portion.
